(19) Europäisches Patentamt
European Patent Office
Office européen des brevets

(11) Publication number: **0 244 051**
**A2**

# (12) EUROPEAN PATENT APPLICATION

(21) Application number: **87300729.8**

(22) Date of filing: **28.01.87**

(51) Int. Cl.[4]: **G02B 6/10**, G02B 5/30, C07C 95/08, C07C 121/70, C07C 87/62, C07C 119/042, C07C 101/453, C07C 97/10, C07C 47/277, C07D 295/06, C07C 103/75

The title of the invention has been amended (Guidelines for Examination in the EPO, A-III, 7.3).

(30) Priority: **04.02.86 GB 8602707**

(43) Date of publication of application: **04.11.87 Bulletin 87/45**

(84) Designated Contracting States: **AT BE CH DE ES FR GB GR IT LI LU NL SE**

(71) Applicant: **IMPERIAL CHEMICAL INDUSTRIES PLC**
**Imperial Chemical House Millbank**
**London SW1P 3JF(GB)**

(72) Inventor: **Allen, Simon**
**8 Madison Avenue**
**Cheadle Hulme Cheshire SK8 5DF(GB)**
Inventor: **Gordon, Paul Francis**
**1 Mill Croft Close**
**Norden Rochdale OL12 7UE(GB)**
Inventor: **Morley, John Oswald**
**9 Winchester Close**
**Bamford Rochdale OL12 5NE(GB)**

(74) Representative: **Draggett, Peter Thornton et al**
**Imperial Chemical Industries PLC P.O. Box 6**
**Bessemer Road**
**Welwyn Garden City Herts AL7 LHD(GB)**

## (54) Non-linear optical elements and compounds therefor.

(57) Optical element comprising a non-centrosymmetric compound comprising an electron donor and an electron acceptor other than one which is or comprises the group -NO linked through a conjugated alka-(polyene)-ylene bridging group containing at least three pairs of conjugated single and double bonds in which the the molecules of the compound are aligned so that the element has a net non-centrosymmetry, a method for their preparation, optical devices comprising such elements, and some of the compounds.

EP 0 244 051 A2

## Non-Linear Optics

This specification describes an invention relating to an optical element and devices based thereon comprising a non-linear optical compound.

According to the present invention there is provided an optical element comprising a non-centrosymmetric compound (hereinafter referred to as "the NLO compound") comprising an electron donor and an electron acceptor linked through a conjugated alka-(polyene)-ylene bridging group containing at least three pairs of conjugated single and double bonds (hereinafter referred to as "the bridging group"), in which the molecules of the NLO compound are aligned so that the the element has a net non-centrosymmetry, which alignment is herein after referred to as "ordered".

A preferred NLO compound can be represented by the formula: A - B - D I

wherein

A represents the electron acceptor;

D represents the electron donor

and B represents the bridging group.

The electron acceptor, A, comprises one or more atoms or groups which readily accept electronic charge, A', preferably containing nitrogen & oxygen atoms, carbon & oxygen atoms, sulphur & oxygen atoms, and/or halogen atoms. Typical examples of A' are $-NO_2$, -CHO, -COOH, halogen, e.g. F, Cl or Br, -COV, -COOW, -CH=NV, -CONVW, -N=NV, $-C\equiv CH$, -CN and -NC where V and W independently represent H or alkyl, alkenyl, heteroaryl or aryl, especially phenyl, and preferably phenyl substituted by one or more electron withdrawing groups, such as $-NO_2$, -CHO, -COOV and -COV. Especially preferred examples of A' are $-NO_2$, -CHO, -COOV, -COV, -CONVW and -CN. The group or groups represented by A' may be attached directly to the bridging group but the electron acceptor, A, preferably also comprises a group containing conjugated double and single bonds, L, through which the group(s) A' are attached to the bridging group. It is preferred that L represents a phenylene or napthylene group, which may carry other substituents, including up to three groups represented by A'. Thus, the electron acceptor, A, can be represented by the formula:

$-(L)_1 -(A')_m$ II

wherein

L and A' are as hereinbefore defined

1 is 0 or 1

and m is an integer from 1 to 3.

Examples of suitable groups of Formula II are 4-cyanophenyl and 2,4-dinitrophenyl.

The electron donor, D, comprises an atom or group which readily gives up an electron, D', which preferably contains a sulphur, nitrogen, oxygen or phosphorus atom carrying one or more hydrogen atoms or hydrocarbon substituents. Examples of suitable groups represented by D' are -OV, -SW, -NVW, -NH-NVW, -NHOH, PVW in which V and W each independently represents H, alkyl, alkenyl, aryl or heteroaryl, or V and W together with the N or P atom to which they are attached represent an hetero-aliphatic or aromatic ring, such as morpholino, piperazino, piperidino and pyridino. Specific examples of suitable groups represented by D' are $OCH_3$, $SCH_3$, $SC_6H_5$, $SC_6H_4N(CH_3)_2$, $NH_2$, $NHCH_3$ and $N(CH_3)_2$.

One or more groups represented by D' may be attached directly to the bridging group or through a linking group, L, comprising a system of conjugated single and double bonds, such as a phenylene or naphthylene group and thus the electron donor, D, can be represented by the formula:

$-(L)_1 -(D')_m$ III

wherein L, D', 1 and n are as hereinbefore defined. Examples of suitable groups of Formula III are 4-(dimethylamino)phenyl and 4-(dodecylamino)-phenyl.

Where the group D' is attached directly to the bridging group it may be monovalent, and attached to the terminal carbon atom of the bridging group through one bond, or bivalent, and attached to the terminal carbon atom of the bridging group through two bonds so as to form a cyclic group incorporating the terminal carbon atom. An example of such an arrangement is:

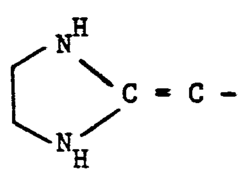

IV

D' B . .

The bridging group, B, may be represented by the formula:

$-CX = CR (-CR = CR)_n -CR = CY - V$

wherein

each R independently represents H or a non-polar substituent;

X represents an electron acceptor, A, or a group R;

Y represents an electron donor, D, or a group R;

and n is from 1 to 28.

The non-polar substituent represented by R is preferably a monovalent hydrocarbon group, especially a $C_{1-4}$-alkyl or $C_{2-4}$-alkenyl group. The bridging group preferably contains up to thirty pairs of conjugated double and single bonds, i.e. n is from 1 to 28, and more preferably up to ten such pairs, i.e. n is from 1 to 8.

Where the groups represented by R at each end of a - C = C -unit in the bridging group are other than hydrogen they may be cis-or trans-, but are preferably trans-, with respect to each other.

Where X represents a second electron acceptor, the two acceptors, which may be the same or different, are attached to the same terminal carbon atom of the bridging group. Likewise, where Y represents a second electron donor, the two donors, which may be the same or different, are both attached to the same terminal carbon atom or the bridging group.

Examples of specific NLO compounds include 1-(4-dimethylamino-phenyl)-6-cyano-6-(octadecoxycarbonyl)-hexa-1,3,5-triene, 1,1-di-(dimethylamino)-6-(2,4-dinitrophenylazo)-hexa-1,3,5-triene, 1-N-methyl-N-dodecylamino-6-(4-nitro phenyl)-hexa-1,3,5-triene and 1,1-(eth-1,2-ylenediamino)-6,6-dicyano-hexa-1,3,5-triene. These and other novel NLO compounds according to Formula I form a further aspect of the present invention.

The molecules of the NLO compound are non-centrosymmetric by virtue of their polarisation along an axis through the the electron donor, D, the bridging group, B, and the electron acceptor, A. A molecule of the compound can therefore be represented as a vector directed along this axis from the substituted electron donor, D, towards the electron acceptor, A. A material, such as an optical element, comprising the NLO compound, either alone or in conjunction with other substances, in which the molecules of the NLO compound are "ordered" (i.e. not randomly oriented so that the sum of the individual molecular vectors is zero) will have an overall non-centrosymmetry and thus be adapted for non-linear optical applications.

The NLO element may comprise the NLO compound of Formula I alone or it may be medium comprising a physical or chemical combination of the NLO compound of Formula I with other compounds which may or may not have NLO properties.

The NLO element may comprise (i) a bulk sample of the NLO compound, such as a single crystal prepared by crystallisation from solution, from the melt, by vapour phase transport or by other known methods of crystallisation, or (ii) an chemically inert medium containing the NLO compound, such as a liquid crystal material, in which the NLO compound may be ordered by the application of a d.c. electric field. The ability of the NLO compound to form an "ordered" crystal is believed to be promoted by the presence in the molecule of a chiral atom which promotes the formation of crystals in which the molecules are "ordered" so that the bulk sample is non-centrosymmetric. It is therefore preferred that an NLO compound for use in the preparation of a single crystal NLO element contains one or more chiral atoms.

Alternatively, the NLO element may comprise a thin film of the NLO compound on a transparent or reflecting substrate, for use in waveguiding geometries well known in this field of work. The film may itself be used as a waveguide, to maximise non-linear optical interactions, or may be used as a non-linear optically-active overcoat to an active or passive waveguide. The formation of an "ordered" film comprising a series of monomolecular layers, by a Langmuir-Blodgett technique, is believed to be promoted by the presence of long chains and thus a preferred class of the NLO compound has a long alkyl or alkenyl chain, preferably as one of the terminal substituents X or Y on the bridging group, or the substituents V or W in one or more of the groups represented by D' and A'.

The film may also be formed, for example, by epitaxial crystal growth or by crystallisation of the material in a narrow cavity between two substrates.

The NLO element may be employed in optical devices which exhibit second-order non linear effects such as second harmonic generation, frequency mixing or the d.c. electro-optical effect.

Examples of non-linear optical effects using an NLO element in accordance with the present invention, in the form of a bulk sample, for example a single crystal, of the NLO compound, include:

(1) Second Harmonic Generation: A laser beam of given frequency, incident on one face of an NLO element comprising an "ordered" single-crystal of the NLO compound, at an angle parallel to the so-called "phase-matching" direction, causes the emission from the element of a coherent beam of laser radiation, at twice the frequency of the incident beam, in a direction substantially parallel to the incident beam.

(2) Electro-optical Amplitude Modulation: A polarised laser beam is directed so that it passes through a birefrequent NLO element, comprising an "ordered" crystal of the NLO compound, at an angle such that the plane of polarisation is rotated, by an angle Q, on passing through the crystal and then through a polarising medium (the 'analyser') which transmits a proportion of the beam corresponding to Q. An electric field, applied across the NLO element causes a change in the birefringence (the "d.c. electro-optic effect") of the element and a consequent change in the angle of

rotation of the polarised output beam, to Q'. The proportion of the beam transmitted by the analyser now corresponds to Q'.

Where the NLO element comprises a Langmuir-Blodgett film of the NLO compound on a substrate this preferably comprises at least two monolayers of the NLO compound, in which the molecules in both layers are "ordered", and more preferably all the molecules are aligned in the same manner and such an optical element comprises a second aspect of the present invention.

By "aligned in the same manner" is meant that the vectors along the axes of polarisation in the molecules are substantially parallel and in the same sense.

It is not essential that the monolayers of the NLO compound are adjacent and it can be advantageous to separate the monolayers with intervening layers of other materials. Where the two monolayers of the NLO compound are adjacent it is preferred that the electron donors, D, of the molecules in one monolayer will be adjacent to the electron acceptors, A, in the adjacent monolayer ("head to tail" array).

Where the substrate is transparent at the wavelength of incident radiation it may be in the form of an optical waveguide on the outer surface of which the NLO compound is deposited. With this form of element an optical signal passing along the waveguide interacts with the superficial coating of the NLO compound, via the evanescent wave which extends into this coating, and gives rise to non-linear optical effects. Examples of suitable substances for a substrate in the form of a waveguide are glass, lithium niobate and silicon nitride on oxidised silicon.

Alternatively, a transparent substrate may be in the form of a plate or disc on one, or both, surfaces of which a coating of the NLO compound can be formed. With this form of element a non-linear optical effect may be obtained by transverse illumination of the substrate and film(s). Suitable substrates for such an optical element include glass, silica and polymethylmethacrylate (PMMA).

Where the substrate is reflecting it conveniently has a plane reflecting surface on which a superficial coating of the present NLO compound may be formed so that the optical signal passes through the coating immediately before and after contact with the reflecting surface. Examples of suitable materials for the reflecting substrate are aluminium, silver, or aluminium or silver films deposited on a support substrate such as glass, silica, quartz or PMMA. With this form of optical element it is possible to attain efficient non-linear processes by exciting the so called "surface plasmon" modes reported in the literature [Stegman et al, Appl.Phys.Lett. 41 (10) 906, 1982; Sand et al, Appl.Optics 21 (22) 3992, 1982].

The optical element in the form of a thin layer of the NLO compound on a substrate may be prepared by a Langmuir-Blodgett technique and according to a third aspect of the invention there is provided a method for the preparation of an optical element having non-linear optical properties which comprises passing a surface of a transparent or reflecting substrate into and out of a Langmuir trough containing a liquid carrying a superficial monomolecular layer of a compound of Formula I (the NLO compound). Where the layers of the NLO compound are not adjacent intervening layers may be formed by passing the substrate into the liquid through a surface carrying a superficial layer of the NLO compound and out of the liquid through another surface carrying a superfical layer of a different compound, or vice versa.

The liquid, hereinafter referred to as the subphase, is preferably an aqueous medium and the mono-molecular layer or layers are maintained in the normal manner by adjustment of the surface area with movable dams.

The optical element comprising a thin layer of the NLO compound on a substrate is adapted for the production second order non-linear optical effects in a number of ways in various optical devices.

According to a further aspect of the present invention there is provided an optical device comprising a non-linear optical element in accordance with the second aspect of the present invention.

An example of an optical device in accordance with this further aspect of the present invention, in which the optical element comprises a substrate in the form of a transparent waveguide having an intimate coating formed by multiple layers of the present NLO compound, consists of an oxidised silicon plate having a first superficial (lower) layer of silicon nitride to form a superficial plane waveguide and a second superficial (upper) layer comprising discrete monolayers of the NLO compound. In operation, a first optical signal is passed through the waveguide, (in the plane of the waveguide) and interacts with the coating, by way of the evanescent wave which extends into the coating. This interaction generates a second optical signal, at the second harmonic frequency with respect to the first optical signal, which can be detected in the combined optical signal leaving the waveguide.

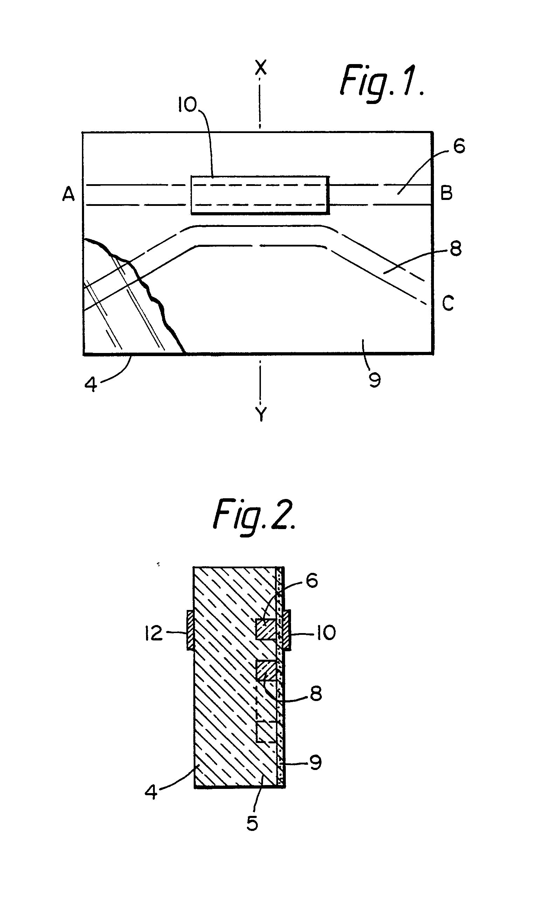

Another device in accordance with the present invention is described in relation to Figures 1 & 2 of the accompanying drawings, in which Figure 1 is a plan view and Figure 2 is a cross-section on the

line X-Y in Figure 1. In the device the optical element comprises a glass substrate, 4, in the upper surface region, 5, of which are two transparent stripe waveguides, 6 & 8, formed in the desired pattern by the well-known ion exchange or ion bombardment techniques. The stripe waveguides are positioned to run closely parallel over the central part of their length during which they are separated by a distance of a few micrometres (typically 2-5 µm). The whole surface of the substrate, 4, is coated with a film, 9, of discrete monolayers of the NLO compound. A pair of electrodes, 10, 12, connected to a power source, not shown, is arranged with one electrode, 10, above and the other, 12 below one of the stripe waveguide, 6. In operation an optical signal is passed through the first waveguide, 6, from A to B and a voltage is applied across the electrodes. This alters the refractive index of the coating, due to the d.c. electro-optic (Pockels) effect, and thus the propagation constant of the first waveguide, 6. By suitable adjustment of the applied voltage the propagation constant of the first waveguide, 6, can be arranged so that the optical signal passing through this waveguide, 6, is coupled into the second waveguide, 8, and produces a second optical signal emerging from the device at C.

The optical element of the second aspect of the present invention may be used in other known forms of optical device incorporating an optical element by replacing the conventional NLO compound used therein, e.g. lithium niobate, with the NLO compound of Formula I.

The compounds in the optical elements of the present invention (including the compounds of formula I) exclude those in which the electron acceptor group A is or comprises the group -NO.

In addition to specific NLO compounds of formula I listed hereinbefore, suitable NLO compounds also include:

1-(4-dimethylaminophenyl)-6-carboxy-6-cyanohexa-1,3,5-triene (1),
1-(4-N-methyl-N-dodecylaminophenyl)-3-methyl-8-formylnona-1,3,5,7-tetraene(2),
1-(4-N-methyl-N-dodecylaminophenyl)-3,8-dimethyl-10-nitrodeca-1,3,5,7,9-pentaene(3),
1-(4-N-methyl-N-dodecylaminophenyl)-6-fluoro-6-cyanohexa-1,3,5-triene,
1-(4-N-methyl-N-dodecylaminophenyl)-6-carboxy-6-cyanohexa-1,3,5-triene (4),
1-(4-N-methyl-N-dodecylaminophenyl)-6-acetyl-6-cyanohexa-1,3,5-triene,
1-(4-N-methyl-N-dodecylaminophenyl)-6-carboxy-6-isocyano-1,3,5-triene,
1-(4-N-methyl-N-dodecylaminophenyl)-6-carboxy-6-nitro-1,3,5-triene,
1-(4-N-methyl-N-dodecylaminophenyl)-20-carboxy-20-cyanoeicosa-1,3,5,7,9,11,13,15,17,19-decaene,
1-(4-N-methyl-N-dodecylaminophenyl)-6-carboxy-6-cyano-3,4-trans-dimethylhexa-1,3,5-triene,
1-(4-dimethylaminophenyl)-3-methyl-8-formylnona-1,3,5,7-tetraene (5),
1-(4-dimethylamino-1-naphthyl)-3-methyl-8-formylnona-1,3,5,7-tetraene,
1-(4-methoxyphenyl)-3-methyl-8-formylnona-1,3,5,7-tetraene,
1-(4-dimethylaminophenyl)-3-methyl-8-carboxynona-1,3,5,7-tetraene,
1-(4-dimethylaminophenyl)-3-methyl-8-fluorononа-1,3,5,7-tetraene,
1-(dimethylaminophenyl)-3-methyl-8-acetylnona-1,3,5,7-tetraene,
1-(4-dimethylaminophenyl)-3-methyl-14-formylpentadeca-1,3,5,7,9,11,13-heptaene,
1-(4-dimethylaminophenyl)-3,(5,6-trans)-trimethyl-8-formylnona-1,3,5,7-tetraene,
1-(4-piperazinophenyl)-3,8-dimethyl-10-nitrodeca-1,3,5,7,9-pentaene,
1-(4-piperidinophenyl)-3,8-dimethyl-10-nitrodeca-1,3,5,7,9-pentaene,
1-(4-N-methyl-N-dodecylaminophenyl)-3,8-dimethyl-10-methoxycarbonyldeca-1,3,5,7,9-pentaene,
1-(4-N-methyl-N-dodecylaminophenyl)-3,8-dimethyl-10-phenyliminomethyldeca-1,3,5,7,9-pentaene,
1-(4-N-methyl-N-dodecylaminophenyl)-3,8-dimethyl-10-dimethylcarbamoyldeca-1,3,5,7,9-pentaene,
1-(4-N-methyl-N-dodecylaminophenyl)-3,8-dimethyl-10-acetyl-11-oxododeca-1,3,5,7,9-pentaene,
1,1-bis-(4-N-methyl-N-dodecylaminophenyl)-3,8-dimethyl-10-formyldeca-1,3,5,7,9-pentaene,
1-(4-dimethylaminophenyl)-3,8-dimethyl-10-carboxy-10-cyanodeca-1,3,5,7,9-pentaene (6),
1-(4-methylthiophenyl)-3,8-dimethyl-10-carboxy-10-cyanodeca-1,3,5,7,9-pentaene,
1-(4-dimethylaminophenyl)-4,7-dimethyl-10-carboxy-10-cyanodeca-1,3,5,7,9-pentaene,
1-(4-dimethylaminophenyl)-3,8-dimethyl-10-phenyldiazo-10-cyanodeca-1,4,5,7,9-pentaene,
1-(4-dimethylaminophenyl)-4,7-dimethyl-10,10-dicarboxydeca-1,3,5,7,9-pentaene,
1-(4-dimethylaminophenyl)-3,8-dimethyl-10-carboxydodeca-1,3,5,7,9-pentaene-11-yne,
1-(4-dimethylaminophenyl)-1-(4-phenylthiophenyl)-3,8-dimethyl-10-carboxy-10-cyanodeca-1,3,5,7,9-pentaene, 1,1-(ethylenediamino)-3,8-dimethyl-10-carboxy-10-cyanodeca-1,3,5,7,9-pentaene,
1-(4-N-methyl-N-dodecylaminophenyl)-3,8-dimethyl-10-carboxy-10-cyanodeca-1,3,5,7,9-pentaene (7),
1-(4-phenylhydrazinophenyl)-3,8-dimethyl-10-carboxy-10-cyanodeca-1,3,5,7,9-pentaene,
1-(4-N-methyl-N-dodecylaminophenyl)-2,9-dimethyl-10-carboxy-10-cyanodeca-1,3,5,7,9-penatene,
1-(4-N-methyl-N-dodecylaminophenyl)-3,8-dimethyl-10,10-dicarboxydeca-1,3,5,7,9-pentaene,
1-(4-N-methyl-N-dodecylaminophenyl)-3,8-dimethyl-10-carboxy-10-(4-nitrophenyl)deca-1,3,5,7,9-pentaene,
1-(4-N-methyl-N-dodecylaminophenyl)-3-prop-1-enyl-8-methyl-10-carboxy-10-cyanodeca-1,3,5,7,9-pentaene,
and
1-(4-N-methyl-N-dodecylaminophenyl)-1,3,8-trimethyl-10-carboxy-10-cyanodeca-1,3,5,7,9-pentaene.

The compounds of formula I may be synthesised analogously to or are readily and routinely derivable from structurally related known compounds. For example the synthesis often comprises at least two of the following steps in any convenient order:

a) reacting a precursor of the electron donor group D with a precursor of the bridging group B (optionally linked to the electron acceptor group A or a precursor thereof) or with a precursor of a part thereof,

b) reacting a precursor of a part of the bridging group B (optionally linked to the electron donor group D or a precursor thereof) with a precursor of another part of the bridging group B (optionally linked to the electron acceptor group A or a precursor thereof,

c) reacting a precursor of the electron acceptor group A with a precursor of the bridging group B (optionally linked to the electron donor group D or a precursor thereof) or with a precursor of part thereof, and

d) where the product is a precursor of the compound of formula I converting that precursor to a compound of formula I.

Synthetic steps of these types are illustrated in the Examples hereinafter. From these it will be seen that steps a) to c) are often conveniently carried out by condensation of a (generally aldehydic) oxo function with a conjugatively unsaturated moiety bearing a good leaving group (e.g. MeO, CN, $Ph_3P^+$) or an activated methylene group (e.g. $^-CH_2NO_2$).

The invention is further illustrated by the following Examples in which all parts and percentages are by weight unless otherwise indicated.

The following Descriptions illustrate the preparation of intermediates for the compounds of the present invention.

## Description 1

### 5-(4-N,N-Dimethylaminophenyl)penta-2,4-dienol- (D1)

Magnesium turnings (1.6g), dry THF (45 ml) and iodine (2 crystals) were stirred and heated whilst ethyl bromide (6ml) in THF (10 ml) was added. Freshly distilled 1-methoxy-but-1-en-3-yne (7.3g) in dry THF (10 ml) was added a 40-45°C and the mixture stirred at room temp for 1 h before cooling to 0°C. 4-N,N-dimethylaminobenzaldehyde (10g) in dry THF (12 ml) was added and the reaction allowed o come to room temperature and stirred for 2 h. After cooling to 0°C lithium aluminium hydride (2g) was added and stirring maintained for 3 h, whereupon water (15 ml) and sulphuric acid (4N, 75 ml) were added. The mixture was separated between toluene and water and the organic layer retained, washed with water and dried. After removal of the solvent the residue was recrystallised from isopropanol to give product (D1) (22.5%). mpt. 150°-153°c; λ $CH_3CN$, 408nm; $m^+$201; ν ($cm^{-1}$) 1650.

## Example 1

### a) 2-cyano-7-(4-N,N-dimethylaminophenyl)hepta-2,4,6-trienoic acid (1)

Aldehyde (D1) (0.5g), cyanoacetic acid (0.3g), ammonium acetate (0.05g) and acetamide (0.05g were mixed in hexane (3ml) and acetic acid (2ml) and the mixture heated at 70°C for 2h. After extraction with ether and removal of solvents the material was recrystallised from toluene-ethanol-hexane to give product (1) (22%) mpt:210° C; $m^+$, 268; λ $CHCl_3$, 489 nm; ν ($cm^{-1}$) 2700-3000 (br), 2210, 1670.

### b) 2,7-Dimethyl-9-(4-N-dodecyl-N-methylamino phenyl) nona-2,4,6,8-tetraene-1-al (2)

Octa-2,4,6-triene-2,6-dial (3.6g) was stirred in DMF (200 ml) with 4-N-methyl-N-dodecylaminophenyltriphenylphosphonium bromide (1.3g) whilst sodium methoxide (70ml,1.1N) was added. After 2 h water was added and the mixture was extracted with toluene, and the organic layer was washed with brine and dried. After removal of solvent the product was purified by column chromatography to give product (2) (2.7g). m,$^+$435; λ DMF 457nm; ν ($cm^{-1}$), 1660.

c) 3,8-Dimethyl-10-(4-N-dodecyl-N-methylamino phenyl)-1-nitrododeca-1,3,5,7,9 pentaene (3)

Aldehyde (2) (2.6g) was heated at 50°C with nitromethane (5ml) and octylamine (0.1ml) for 2 h. The material was subjected to column chromatography and recrystallised from hexane to give (3) (12.4%) m, $^{+}$478; λ ($cm^{-1}$) 1580, 1380: ν $CHCl_3$ 526 nm.

The compounds of formula I listed hereinbefore are prepared analogously to compounds (1) to (3) in this Example 1, in particular compounds (4) to (7) listed hereinbefore.

Example 2

A dipping bath was prepared by slowly dripping 10 ul of a solution of compound (4) in chloroform (1mg/ml) from a micro-syringe onto the surface of an aqueous sub-phase having a surface area of 1000 $cm^2$ in a Langmuir trough. The surface pressure was adjusted to 15 mN/m by movement of the barriers and maintained at this level throughout the dipping process.

A pre-cleaned, thin glass plate was successively dipped into and withdrawn from the sub-phase at a speed of 3 mm/min. Deposition of a monolayer of (4) occurred substantially only during withdrawal of the substrate from the sub-phase and dipping was continued until a film comprising 20 monolayers of (4) had been deposited on the part of both of the parallel faces of the plate which passed through the monolayer of (4). All the molecules of (4) in the film were aligned substantially parallel with the vectors in the same sense, i.e. the molecules in the separate monolayers were in "head to tail" array. The film of (4) was removed from one plane surface of the plate, and the resulting optical element comprised a glass substrate coated on one plane surface with multiple monolayers of (4).

Elements comprising compounds of formula I listed hereinbefore are prepared analogously to that above, in particular elements comprising compounds (6) and (7) listed hereinbefore.

Example 3

The optical element described in Example 2 was used in the following manner to demonstrate the non-centro-symmetric nature of the applied film.

A beam of light from a Nd:YAG pulsed layer (wavelength: 1.06 um) was passed transversely through the plate and the film of (4). The intensity of light at the second harmonic (wavelength: 0.53 um) generated during passage through the element was detected and measured with a photodiode. This measurement was used to calculate the non-linear optical figure of merit, $(x^{(2)})^2/n^3$, in which $x^{(2)}$is the second order non-linear optical coefficient and n is the refractive index of th NLO compound (4).

The measured figure of merit was 14 times that for quartz at the same wavelength.

Other elements referred to in Example 2 are tested analogously.

Example 4

The second order molecular hyperpolarisability coefficient (β) of the compounds of formula I, including compounds (3) and (5), were determined routinely using the well-known EFISH (Electric Field induced Second Harmonic ) experiment described in J. Chem. Phys., 63, 2666 (1975) on a number of solutions of the compounds at various concentrations to determine the macroscopic susceptibility (see also J. Chem. Phys., 67 446 (1979) and Phys, Rev Lett. 8 21 (1962)) and using routinely determined refractive indices and dielectric constants.

Example 5

An approximate measure of the relative efficiencies of second harmonic generation in potential nonlinear optical materials can be obtained using the powder SHG technique first described by Kurtz and Perry (J. Appplied Physics, Vol. 39, p. 3798, 1968). A polycrystalline sample of the material of interest is first ground in a mortar and pestle to produce an approximately uniform particles size and a standard sized cell (19 mm diameter x 1.5 mm deep) is filled with this powder. The powder cell is mounted in the path of a Nd:YAG laser beam. The fundamental wavelength of the laser is 1.06 µm, but alternatively an incident wavelength of 1.9 µm can be obtained by passing the 1.06 beam through a Hydrogen filled raman cell prior to the sample. The longer wavelength radiation is used for samples which strongly absorb light at 530 nm (i.e. at the second harmonic of the 1.06 beam). In this case the true efficiency of the materials could not be determined with the 1.06 µ radiation as the second harmonic produced is reabsorbed by the material.

The laser beam (at 1.06 or 1.9 µm) is incident on the powder sample, and may result in the generation of light at the second harmonic frequency (532 nm or 950 nm respectively). The amount of harmonic generated gives a measure of the nonlinear optical efficiency of the material. The

second harmonic radiation is detected either in reflection or alternatively in transmission, with the photodetector placed directly behind the sample. The sample is then replaced with a cell containing powder of a standard material (usually urea) and the experiment repeated. Comparison of the signals obtained in the two cases determines the efficiency of the material of interest relative to urea.

Typically, an incident laser energy of about 1 mJ per pulse is used, the pulse being of about 10 nsec duration, with a repetition rate of 10 Hz.

## Claims

1. An optical element comprising a non-centrosymmetric compound comprising an electron donor and an electron acceptor other than one which is or comprises the group -NO linked through a conjugated alka-(polyene)-ylene bridging group containing at least three pairs of conjugated single and double bonds, in which the the molecules of the compound are aligned so that the element has a net non-centrosymmetry.

2. An optical element according to claim 1 wherein the compound is of the formula:

A-B-D I

wherein

A represent the electron acceptor;

B represent the bridging group; and

D represents the electron donor and is of the formula $-(L)_1-(D')_m$ II

wherein

1 is 0 or 1;

m is 1, 2 or 3.

L is a benzene or naphthalene nucleus;

D' is selected from -OV, -SW, -NVW, -NH-NVW, -NHOH, PVW in which V and W each independently represents H, alkyl, alkenyl, aryl or heteroaryl, or V & W together with the N atom to which they are attached represent morpholino, piperazino, piperidino or pyridino; or

when 1 is 0, D is attached to a terminal carbon atom of the bridging group B and also by a second bond so as to form a cyclic group incorporating the terminal carbon atom.

3. An optical element according to claim 2 wherein the compound is of the formula

A-B-D I

wherein

D represents the electron donor;

B represents the bridging group; and

A represents the electron acceptor and is of the formula:

$-(L-_1-(A')_m$ II

wherein

L is a benzene or naphthalene nucleus;

A' is selected from F, Cl or Br, -COV, -COOW, -CH=NV, -CONVW, -N=NV, -C=CH, -CN and NC where V and W independently represent H or alkyl, alkenyl, heteroaryl or phenyl optionally substituted by one or more electron withdrawing groups selected from $-NO_2$, -CHO, -COOV and -COV.

4. An optical element according to claim 1, wherein the compound is of the formula:

A-B-D I

wherein

D represents the electron donor;

A represents the electron acceptor; and

B represents the bridging group and is of the formula

$-CX=CR(-CR=CR)_n-CR=CY-V$

wherein

each R independently represents H or a $C_{1-4}$ alkyl or $C_{2-4}$ alkenyl group;

X represents a second electron acceptor A which may be the same as or different from the acceptor A in the formula I, or a group R as defined above;

Y represents a second bond to the electron donor D in formula I which forms a cyclic group with the terminal carbon atom bearing D and Y, a second electron donor D which may be the same as or different from the donor D in formula I, or a group R as defined above.

5. An optical element according to claim I comprising a single crystal of a compound of formula I which contains one or more chiral atoms, or a chemically inert liquid crystal material containing a compound of formula I which is aligned as defined in claim 1 by the application of a d.c. electric field.

6. An optical element according to claim 1 wherein the element comprises at least two monolayers of the compound of formula I in each of which monolayers the molecules of the compound are aligned as defined in claim 1.

7. An optical element according to claim 6 wherein all the molecules of the compound of formula I are aligned such that the vectors along the axis of polarisation in the molecules are substantially parallel and in the same sense.

8. A method for the preparation of an optical element according to claim 6 which comprises passing a surface of a transparent or reflecting substrate into and out of a Langmuir trough containing a liquid carrying a superficial monomolecular layer of a compound of formula I.

9. An optical device comprising a non-linear optical element according to claim 6.

10. A compound of formula I as defined in claim 2.

Fig. 1.

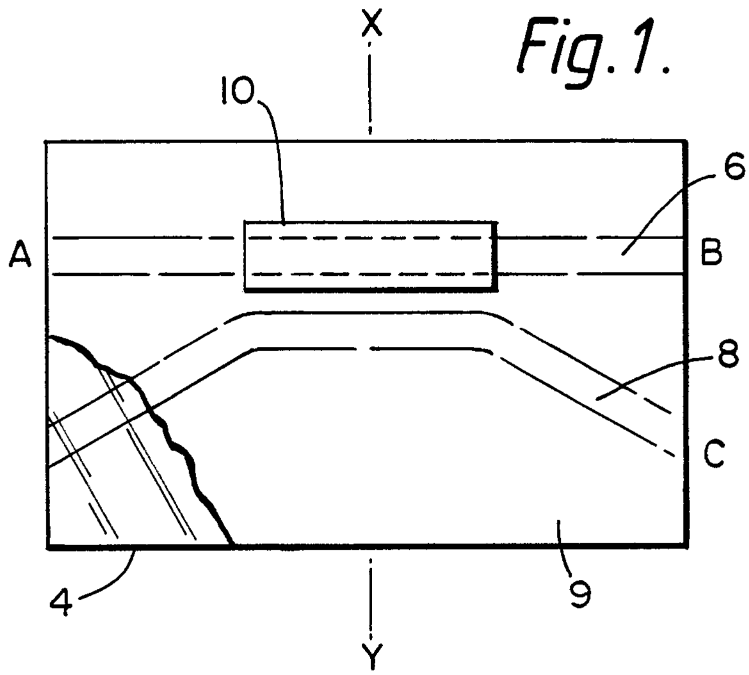


Fig. 2.